# EUROPEAN PATENT APPLICATION

(11) **EP 3 466 397 A1**
(43) Date of publication of application: **10.04.2019**
(21) Application number: 17806222.0
(22) Date of filing: 20.04.2017
(51) Int. Cl.: A61K 6/10, A61C 13/34

(54) **METHOD FOR KNEADING DENTAL PLASTER POWDER**

(30) Priority: 30.05.2016 JP 2016107022
(71) Applicant: GC Corporation, Sunto-gun, Shizuoka 410-1307 (JP)
(72) Inventor: MORI, Daizaburo, Tokyo 174-8585 (JP); YOSHINAGA, Masatoshi, Tokyo 174-8585 (JP); WATANABE, Kaori, Tokyo 174-8585 (JP)
(74) Representative: Müller-Boré & Partner Patentanwälte PartG mbB
(86) International application number: PCT/JP2017/015917
(87) International publication number: WO 2017/208662

(57) **Abstract**

A method for kneading a dental gypsum powder is provided that includes putting the dental gypsum powder and water in a bottle, the dental gypsum powder containing gypsum hemihydrate and a polycarboxylate-based water reducing agent, contained at 0.05 parts by mass to 0.8 parts by mass with respect to 100 parts by mass of the gypsum hemihydrate, sealing the bottle, and shaking the bottle.

## Description

### TECHNICAL FIELD

The present invention relates to a method for kneading a dental gypsum powder.

### BACKGROUND ART

In recent years, there is growing interest in home-visit medical care as a promising medical service model for meeting the needs of the ageing population, and the number of home visits being made is increasing. The demand for home-visit medical care is expected to increase further in the future. The same holds true for dental care, and opportunities for dentists to make home visits are also expected to increase in the future.

One type of operation that may be performed in a home visit for dental care includes the fabrication of a gypsum model (working model or study model) reproducing the condition of the oral cavity of a patient. In this operation, an impression of the oral cavity of a patient is taken in advance using a dental impression material to obtain a negative imprint, a dental gypsum powder and water are kneaded to obtain a gypsum slurry, the gypsum slurry is poured into the negative imprint, and a gypsum model is fabricated by causing the gypsum slurry to set.

Typically, in a dental clinic setting or a dental laboratory setting, the operation of kneading a dental gypsum powder and water to obtain a gypsum slurry involves putting a predetermined amount of powder of a dental gypsum composition and water for kneading in a small rubber bowl and kneading the powder and water using a dedicated spatula such as a gypsum spatula. Because a large number of air bubbles are formed at the time of kneading, a mechanical device, such as a vibrator or a vacuum stirrer, or a separate defoamer has to be used to prevent air bubbles from mixing into the gypsum slurry (see, e.g., Patent Document 1).

### PRIOR ART DOCUMENTS

### PATENT DOCUMENTS

Patent Document 1: Japanese Unexamined Patent Publication No. H06-178926

### SUMMARY OF THE INVENTION

### PROBLEM TO BE SOLVED BY THE INVENTION

However, in a home visit setting, the kneading operation is performed at an ordinary household. Because an ordinary household does not have a mechanical device such as a vibrator, a heavy and bulky mechanical device such as a vibrator has to be carried to the household in order to obtain a gypsum slurry without air bubbles. Such a requirement imposed a heavy burden on transportation. Also, in addition to the vibrator, many other tools such as a rubber bowl and a gypsum spatula have to be readily available such that preparation for the kneading operation may be quite complicated.

In this respect, an aspect of the present invention is directed to providing a method for kneading a dental gypsum powder to easily obtain a gypsum slurry without air bubbles.

### MEANS FOR SOLVING THE PROBLEM

To solve the above problems of the related art, the inventors of the present invention conducted extensive research and experimentation. As a result, the inventors found that a gypsum slurry without air bubbles can be easily obtained by putting a dental gypsum powder having a specific composition and water in a bottle, sealing the bottle, and shaking the bottle. The inventors have thus conceived the present invention.

According to one embodiment of the present invention, a method for kneading a dental gypsum powder is provided that includes putting the dental gypsum powder and water in a bottle, the dental gypsum powder containing gypsum hemihydrate and a polycarboxylate-based water reducing agent, contained at 0.05 parts by mass to 0.8 parts by mass with respect to 100 parts by mass of the gypsum hemihydrate, sealing the bottle, and shaking the bottle.

### ADVANTAGEOUS EFFECT OF THE INVENTION

By implementing the method for kneading a dental gypsum powder according to an embodiment of the present invention, a gypsum slurry without air bubbles can be easily obtained without using a mechanical device such as a vibrator.

### EMBODIMENTS FOR IMPLEMENTING THE INVENTION

In the following, embodiments for implementing the present invention will be described. Note, however, that the present invention is not limited to the embodiments described below and various modifications and substitutions may be made with respect to the embodiments described below without departing from the scope of the present invention.

A method for kneading a dental gypsum powder according to an embodiment of the present invention includes steps of putting a dental gypsum powder (described below) and water in a bottle, sealing the bottle, and shaking the bottle.

The bottle used in the present embodiment includes an opening and closing means. The closing means is for closing and sealing the opening of the bottle after putting the dental gypsum powder and water in the bottle via the opening.

Note that when "sealing" the bottle, the seal has to be sufficiently airtight to prevent the dental gypsum powder and water from leaking outside when the bottle is shaken after the dental gypsum powder and water have been put in the bottle and the opening of the bottle has been closed. However, any higher level of airtightness is not required.

The inner diameter of the opening of the bottle is not particularly limited as long as the dental gypsum powder and water can pass therethrough and a gypsum slurry can be discharged therefrom. For example, the inner diameter of the opening may preferably be greater than or equal to 1 cm and less than or equal to 20 cm.

The closing means is not particularly limited as long as the closing means is capable of preventing the dental gypsum powder and water from leaking outside when the bottle is shaken after the dental gypsum powder and water have been put in the bottle and the opening of the bottle has been closed. For example, the closing means may be integrated with the bottle, or the closing means may be a part that is separate from the bottle. An example of the closing means that is integrated with the bottle includes a shutter mechanism or the like. Examples of a part that is separate from the bottle include a lid, a clip, and the like. Example methods of attaching the lid to the bottle include screwing, fitting, pushing, capping, and the like.

Also, in some embodiments, the closing means may be configured to close the bottle upon being applied a force by a user when shaking the bottle. For example, in a case where the closing means has a folding configuration for closing the opening of the bottle by being folded, the opening of the bottle may be closed by having the user apply a force to the closing means so as to maintain the closing means in the folded state when shaking the bottle.

Although the capacity of the bottle is not particularly limited, the capacity of the bottle is preferably greater than or equal to 0.01 L and less than or equal to 1 L in consideration of the required amount of the gypsum slurry to be prepared in one kneading operation during a home visit.

In the present embodiment, the user shakes the bottle, causing the dental gypsum powder and water to collide with the inner wall of the bottle, thereby kneading the dental gypsum powder. In this respect, the bottle preferably has sufficient strength to withstand deformation upon being shaken. Note that sufficient strength to withstand deformation means a strength that is sufficient to maintain its overall shape when the user shakes the bottle. The bottle may be a container such as a jar or a vial, for example.

Example materials of the bottle include glass, rubber, resin, metal, and the like. Preferred examples of resin that may be used include polyethylene resin, ethylene vinyl acetate resin, polypropylene resin, vinyl chloride resin, polystyrene resin, acrylonitrile-styrene resin, acrylonitrile-butadiene-styrene resin, polyethylene terephthalate resin, acrylic resin, polyvinyl alcohol resin, polyvinylidene chloride vinylidene resin, polycarbonate resin, polyamide resin, polyacetal resin, polybutylene terephthalate resin, fluorocarbon resin, phenol resin, melamine resin, urea resin, polyurethane resin, epoxy resin, unsaturated polyester resin, and composite resins of the above resins.

The wall of the bottle has to be sufficiently thick to withstand deformation. For example, the wall thickness of the bottle may preferably be greater than or equal to 0.1 mm and less than or equal to 1 cm. The wall thickness may more preferably be greater than or equal to 1 mm and less than or equal to 8 mm.

Also, in a preferred embodiment, at least a part of the wall of the bottle is transparent or translucent so that its interior may be visible.

The bottom surface of the bottle is preferably flat so that the bottle can stand upright. Also, the opening of the bottle is preferably disposed on the opposite side of the bottom surface of the bottle.

The weight of the bottle is preferably less than or equal to 500 g so that the user can easily hold and shake the bottle by hand.

As for the method of shaking the bottle, the user may hold the bottle with his/her hand and shake the bottle, or the user may shake the bottle using a tool or device suitable for the bottle, for example.

Although the time period for shaking the bottle is not particularly limited, the bottle may preferably be shaken for a time period from 30 seconds to 5 minutes, for example.

The bottle may be cleaned after use and reused, for example. Also, to omit the need to wash the bottle, the bottle may be for single use, for example.

The dental gypsum powder used in the present embodiment is a dental gypsum powder containing gypsum hemihydrate and a polycarboxylate-based water reducing agent, contained at 0.05 parts by mass to 0.8 parts by mass with respect to 100 parts by mass of the gypsum hemihydrate. By using the dental gypsum powder according to the present embodiment, a gypsum slurry without air bubbles may be easily obtained by merely putting the dental gypsum powder and water in a bottle, sealing the bottle, and shaking the bottle.

Examples of the gypsum hemihydrate include α-gypsum hemihydrate, β-gypsum hemihydrate, and a mixture of α-gypsum hemihydrate and β-gypsum hemihydrate.

Examples of the polycarboxylate-based water reducing agent include polycarboxylate ether; water soluble salt of a copolymer of an open-chain olefin having 5 or 6 carbon atoms and ethylenically unsaturated carboxylic acid anhydride; a copolymer of polyethylene glycol monoallyl ether and an unsaturated dicarboxylic acid; a copolymer of polyalkylene glycol mono (meth)acrylic acid ester and (meth)acrylic acid; a copolymer of (meth)acrylic acid amide having a sulfone group at a terminal, acrylic acid ester, and (meth)acrylic acid, a copolymer of a monomer having a sulfone group, such as vinyl sulfonate, aryl sulfonate, methacryl sulfonate, (meth)acrylic acid, and some other monomer; a quaterpolymer of a monomer having a sulfone group at a terminal, polyalkylene glycol mono (meth)acrylic acid ester, and a polyalkylene glycol mono (meth)acrylic acid ether, and (meth)acrylic acid.

The content of the polycarboxylate-based water reducing agent is greater than or equal to 0.05 parts by mass and less than or equal to 0.8 parts by mass with respect to 100 parts by mass of the gypsum hemihydrate, and preferably greater than or equal to 0.15 parts by mass and less than or equal to 0.3 parts by mass with respect to 100 parts by mass of the gypsum hemihydrate. The content of the polycarboxylate-based water reducing agent is more preferably greater than or equal to 0.15 parts by mass and less than or equal to 0.25 parts by mass with respect to 100 parts by mass of the gypsum hemihydrate. When the content of the polycarboxylate-based water reducing agent is less than 0.05 parts by mass with respect to 100 parts by mass of the gypsum hemihydrate, the above-described effect of the present embodiment cannot be sufficiently obtained and air bubbles will be generated. On the other hand, when the content of the polycarboxylate-based water reducing agent is greater than 0.8 parts by mass with respect to 100 parts by mass of the gypsum hemihydrate, the flowability of the obtained gypsum slurry will not increase, the strength of the gypsum body resulting from letting the gypsum slurry set will decrease, and the setting time will be prolonged.

The dental gypsum powder preferably further contains gypsum dihydrate. By including gypsum dihydrate in the dental gypsum powder, the setting time of the gypsum slurry may be accelerated. The content of the gypsum dihydrate is preferably greater than or equal to 2 parts by mass and less than or equal to 4 parts by mass with respect to 100 parts by mass of the gypsum hemihydrate, and more preferably greater than or equal to 2 parts by mass and less than or equal to 3.5 parts by mass with respect to 100 parts by mass of the gypsum hemihydrate. The content of the gypsum dihydrate is more preferably greater than or equal to 2 parts by mass and less than or equal to 3 parts by mass with respect to 100 parts by mass of the gypsum hemihydrate. When the content of the gypsum dihydrate is greater than or equal to 2 parts by mass with respect to 100 parts by mass of the gypsum hemihydrate, the setting time of the gypsum slurry may be sufficiently accelerated. When the content of the gypsum dihydrate is less than or equal to 4 parts by mass with respect to 100 parts by mass of the gypsum hemihydrate, the flowability of the gypsum slurry may be prevented from decreasing and the setting expansion of the gypsum slurry may be prevented from increasing. Thus, a gypsum model can be fabricated with higher accuracy.

Examples of the gypsum dihydrate include natural gypsum and synthetic gypsum. An example of synthetic gypsum includes gypsum newly synthesized from sulfuric acid and calcium carbonate, but most synthetic gypsum is obtained as a byproduct of various chemical processes (byproduct gypsum). The average particle diameter of the synthetic gypsum may be from approximately 30 µm to 60 µm, but gypsum dihydrate containing crystals having an average particle diameter that is greater than 60 µm may also be used.

The dental gypsum powder preferably further contains potassium sulfate. By including potassium sulfate in the dental gypsum powder, the setting expansion of the dental gypsum powder may be controlled. In the case of including potassium sulfate in the dental gypsum powder, the content of potassium sulfate is preferably greater than or equal to 0.5 parts by mass and less than or equal to 3 parts by mass with respect to 100 parts by mass of the gypsum hemihydrate. When the content of potassium sulfate is greater than or equal to 0.5 parts by mass with respect to 100 parts by mass of the gypsum hemihydrate, the setting expansion of the dental gypsum powder may be sufficiently controlled, and when the content of potassium sulfate is less than or equal to 3 parts by mass with respect to 100 parts by mass of the gypsum hemihydrate, excessive setting acceleration of the dental gypsum powder may be controlled. The content of potassium sulfate is more preferably greater than or equal to 0.5 parts by mass and less than or equal to 2 parts by mass.

Note that the content of potassium sulfate in the dental gypsum powder may also be defined with respect to the content of the gypsum dihydrate. Specifically, by setting the content of potassium sulfate to be greater than or equal to 25 mass% and less than or equal to 100 mass% with respect to the content of the gypsum dihydrate, suitable setting expansion for a gypsum model may be obtained. Thus, the content of potassium sulfate is preferably adjusted to be within the above range.

The dental gypsum powder may further contain a setting expansion inhibitor such as sodium sulfate or potassium tartrate; a coloring agent; a weight reducing agent; and/or any known setting retardant including a salt such as citrate, borate, or acetate; or a water-soluble polymer such as a starch, gum arabic, carboxymethyl cellulose, gelatin or the like.

The mix ratio of dental gypsum powder and water in the method of kneading a dental gypsum powder according to the present embodiment is as follows. Specifically, the mass ratio of water to dental gypsum powder is preferably greater than or equal to 0.18 and less than or equal to 0.6. When the mass ratio of water to dental gypsum powder is greater than or equal to 0.18, the flowability of the gypsum slurry may be enhanced, and when the mass ratio of water to dental gypsum powder is less than or equal to 0.6, the strength of the gypsum body resulting from letting the gypsum slurry set may be enhanced. The mass ratio of water to dental gypsum powder is more preferably greater than or equal to 0.18 and less than or equal to 0.4. The mass ratio of water to dental gypsum powder is more preferably greater than or equal to 0.18 and less than or equal to 0.3.

### [Examples]

In the following, the method for kneading a dental gypsum powder according to the present invention will be described in detail with reference to specific examples. Note, however, that the present invention is not limited to these specific examples.

### <Preparation of Dental Gypsum Powder>

Raw materials shown in Table 1 below were put in a pot mill, and the raw materials were mixed for 60 minutes to prepare a dental gypsum powder according to the examples described below.

Water reducing agents (see Table 1) used in the examples are described below.

"Melflux 2651F", "Melflux 5581F", "Melflux 4930F", and "Melflux 6681F" are all polycarboxylate-based water reducing agents manufactured by SKW East Asia Co., Ltd.

"Mighty 100", manufactured by Kao Corporation, is a water reducing agent composed mainly of sodium salt of naphthalenesulfonic acid formaldehyde condensate.

"Melment F10M", manufactured by SKW East Asia Co., Ltd., is a melamine sulfonic acid-based water reducing agent composed mainly of melamine resin sulfonic acid formalin condensate.

Six different kneading examples (Examples 1 to 6) for kneading a dental gypsum powder were prepared. In these six kneading examples, the dental gypsum powder contained gypsum hemihydrate and 0.05 to 0.8 parts by mass of a polycarboxylate-based water reducing agent with respect to 100 parts by mass of the gypsum hemihydrate, and a mass ratio of water to dental gypsum powder was set to be greater than or equal to 0.18 and less than or equal to 0.6. For purposes of comparison, kneading examples containing no polycarboxylate-based water reducing agent (Comparative Examples 1, 6 and 7), kneading examples containing a polycarboxylate-based water reducing agent at a lower content than the above content range (Comparative Examples 2 and 4), a kneading example containing a polycarboxylate-based water reducing agent at a higher content than the above content range (Comparative Example 3), and a kneading example in which the mass ratio of water to dental gypsum powder was set to be greater than the above mass ratio range (Comparative Example 5) were prepared.

### <Kneading Dental Gypsum Powder>

100 g of the prepared dental gypsum powder and water at the corresponding mass ratio shown in Table 1 were put in a cylindrical bottle made of polystyrene resin (opening inner diameter: about 4 cm; height: about 7 cm; capacity: about 90 mL; weight: about 25 g; wall thickness: about 1.5 mm) after which the bottle was sealed, and the bottle was shaken manually for 30 seconds to obtain a gypsum slurry.

### <Presence/Absence of Air Bubbles>

The obtained gypsum slurry was poured into an impression material, and after 5 minutes had elapsed, the resulting gypsum model (gypsum body) was removed from the impression material. The obtained gypsum model was visually evaluated for the presence/absence of air bubbles. The results of the evaluation are shown in Table 1.

### <Strength of Gypsum Body>

With respect to the gypsum model (gypsum body) obtained by the above method, after 60 minutes had elapsed from the time kneading of the dental gypsum powder was started, the physical property (strength) of the resulting gypsum body was evaluated using the test method specified in JIS T6605 "Dental Stone". The results of the evaluation are shown in Table 1. The rating scale categories used for the strength evaluation in Table 1 are described below.

| | |
|---|---|
| Excellent | strength is greater than 10 MPa |
| Good | strength is greater than or equal to 3 MPa and less than or equal to 10 MPa |
| Fair | strength is less than 3 MPa |

**[Table 1]**

| (Composition: Parts by weight) | | | | | | | | | | | | | | | |
|---|---|---|---|---|---|---|---|---|---|---|---|---|---|---|---|
| | | | **Example 1** | **Example 2** | **Example 3** | **Example 4** | **Example 5** | **Example 6** | **Comparative Example 1** | **Comparative Example 2** | **Comparative Example 3** | **Comparative Example 4** | **Comparative Example 5** | **Comparative Example 6** | **Comparative Example 7** |
| **Gypsum hemihydrate** | *α*-Gypsum hemihydrate | | 100 | 100 | 100 | 100 | 100 | 100 | 100 | 100 | 100 | 100 | 100 | 100 | 100 |
| **Polycarboxy late-based water reducing agent** | Polycarboxylate ether | Melflux 2651F | 0.3 | | | | 0.6 | 0.3 | | 0.01 | 0.9 | | | | |
| | | Melflux 5581F | | 0.2 | | | | | | | | | 0.1 | | |
| | | Melflux 4930F | | | 0.1 | | | | | | | 0.05 | | | |
| | | Melflux 6681F | | | | 0.5 | | | | | | | | | |
| **Miscellaneous** | Gypsum dihydrate | | 3 | 3 | 4 | 2.5 | 3.4 | 3 | | 1 | | | | 3 | 3 |
| | Potassium sulfate | | | | 2 | 0.6 | | | 0.1 | | | 0.5 | | | |
| | Sodium sulfate | | 0.6 | 1 | | | 0.6 | 0.6 | | | | | | 0.6 | 0.6 |
| **Water reducing agent other than polycarboxylate-based water reducing agent** | | Mighty 100 | | | | | | | | | | | | 0.1 | |
| | | Melment F10M | | | | | | | | | | | | | 0.1 |
| **Mass ratio of water to dental gypsum powder** | | | 0.24 | 0.3 | 0.4 | 0.42 | 0.2 | 0.45 | 0.3 | 0.24 | 0.24 | 0.16 | 0.7 | 0.3 | 0.3 |
| **Presence/absence of air bubbles** | | | NO | NO | NO | NO | NO | NO | YES | YES | NO | YES | NO | YES | YES |
| **Strength of gypsum body (after 60 min)** | | | excellent | excellent | good | good | excellent | good | excellent | excellent | fair | excellent | fair | excellent | excellent |

Referring to the evaluation results shown in Table 1, it can be appreciated that when the content of the polycarboxylate-based water reducing agent was greater than or equal to 0.05 parts by mass and less than or equal to 0.8 parts by mass with respect to 100 parts by mass of the gypsum hemihydrate, air bubbles were absent in the gypsum slurry that had set, and a gypsum body having sufficient strength was obtained.

On the other hand, when no polycarboxylate-based water reducing agent was included (Comparative Examples 1, 6, and 7), or when the content of the polycarboxylate-based water reducing agent was lower than the above content range (Comparative Examples 2 and 4), air bubbles were observed in the gypsum slurry that had set. Further, when the content of the polycarboxylate-based water reducing agent was higher than the above content range (Comparative Example 3), no air bubbles were observed in the gypsum slurry that had set, but the strength of the resulting gypsum body was insufficient.

Also, even when the content of the polycarboxylate-based water reducing agent was within the above content range, when the mass ratio of water to dental gypsum powder was greater than the above mass ratio range (Comparative Example 5), the strength of the resulting gypsum body was insufficient.

As described above, the content of the polycarboxylate-based water reducing agent and the mass ratio of water to dental gypsum powder were altered, and the presence/absence of air bubbles in the resulting gypsum slurry and the strength of the gypsum body resulting from letting the gypsum slurry set were evaluated. As a result, it was found that a gypsum slurry without air bubbles can be obtained when the content of the polycarboxylate-based water reducing agent is greater than or equal to 0.05 parts by mass and less than or equal to 0.8 parts by mass with respect to 100 parts by mass of the gypsum hemihydrate, and the mass ratio of water to dental gypsum powder is less than or equal to 0.6.

### INDUSTRIAL APPLICABILITY

The present invention relates to a method for kneading a dental gypsum powder and may be suitably implemented as a method for kneading a dental gypsum powder in a home visit setting or some environment not requiring a dedicated mechanical device or tool, for example.

The present application is based on and claims priority to Japanese Patent Application No. 2016-107022 filed on May 30, 2016, the entire contents of which are hereby incorporated by reference.

## Claims

1. A method for kneading a dental gypsum powder, the method comprising:
putting the dental gypsum powder and water in a bottle, the dental gypsum powder containing gypsum hemihydrate and a polycarboxylate-based water reducing agent, contained at 0.05 parts by mass to 0.8 parts by mass with respect to 100 parts by mass of the gypsum hemihydrate, sealing the bottle, and shaking the bottle.

2. The method for kneading a dental gypsum powder according to claim 1, wherein a mass ratio of the water to the dental gypsum powder is greater than or equal to 0.18 and less than or equal to 0.6.
